**Europäisches Patentamt**

**European Patent Office**

**Office européen des brevets**

(11) Numéro de publication: **0 266 281 B1**

(12) **FASCICULE DE BREVET EUROPEEN**

(45) Date de publication du fascicule du brevet:
**01.08.90**

(21) Numéro de dépôt: **87420264.1**

(22) Date de dépôt: **30.09.87**

(51) Int. Cl.⁵: **C07C 19/08, C07C 17/20**

(54) **Procédé de préparation d'iodure de trifluorométhyle.**

(30) Priorité: **10.10.86 FR 8614283**

(43) Date de publication de la demande:
**04.05.88 Bulletin 88/18**

(45) Mention de la délivrance du brevet:
**01.08.90 Bulletin 90/31**

(84) Etats contractants désignés:
**AT BE CH DE ES FR GB GR IT LI LU NL SE**

(56) Documents cités:
**Neant**

**Le dossier contient des informations techniques présentées postérieurement au dépôt de la demande et ne figurant pas dans le présent fascicule.**

(73) Titulaire: **RHONE-POULENC CHIMIE, 25, quai Paul Doumer, F-92408 Courbevoie Cédex(FR)**

(72) Inventeur: **Tordeux, Marc, 1, rue Seignelay, F-92330 Sceaux(FR)**
Inventeur: **Wakselman, Claude, 5, rue Chaneze, F-75016 Paris(FR)**

(74) Mandataire: **Le Pennec, Magali et al, RHONE-POULENC INTERSERVICES Service Brevets Chimie 25, Quai Paul Doumer, F-92408 Courbevoie Cédex(FR)**

## Description

La présente invention concerne un procédé de préparation d'iodure de trifluorométhyle. Elle concerne plus particulièrement un procédé de transformation du bromure de trifluorométhyle en iodure de trifluorométhyle.

En effet, l'iodure de trifluorométhyle est un produit actuellement extrêmement coûteux alors que son analogue bromé l'est peu.

Les procédés actuellement connus de préparation de l'iodure de trifluorométhyle sont au nombre de quatre.

Le premier décrit par BANKS, EMELEUS, HASZELDINE et KERRIGAN dans le Journal of Chemical Society p 2188 (1948) consiste à faire réagir le tétraiodure de carbone avec le pentafluorure d'iode trente minutes à 90-100°C. Ce procédé est inutilisable dans l'industrie car le tétraiodure de carbone n'est pas disponible.

Le second procédé de préparation de l'iodure de trifluorométhyle décrit par HASZELDINE dans le Journal of Chemical Society p. 584 (1951) consiste à faire réagir le trifluoroacétate d'argent avec de l'iode. Les rendements annoncés sont très bons (91%) mais le coût et la toxicité du trifluoroacétate d'argent empêchent d'utiliser ce procédé au niveau industriel.

Le troisième procédé de préparation de CF$_3$I décrit par KRESPAN dans le Journal of Organic Chemistry 23 (1958) page 2016 consiste à faire réagir le chlorure de trifluoroacétyle avec l'iodure de potassium à 200°C pendant 6 heures pour un rendement global de réaction ne dépassant pas 30 %. Le chlorure de trifluoroacétyle est un gaz cher, les rendements sont médiocres, ce qui rend ce procédé trop coûteux pour une exploitation industrielle.

Le quatrième procédé de préparation de CF$_3$I décrit par PASKOVICH, GASPAR et HAMMOND dans le Journal of Organic Chemistry 32 (1967) page 833 consiste à faire réagir le trifluoroacétate de sodium avec l'iode en suspension dans le diméthylformamide et au reflux de ce dernier c'est à dire à une température d'environ 140°C. L'utilisation du diméthylformamide au reflux, en présence d'iode, en milieu aprotique est à éviter dans l'industrie pour des problèmes de sécurité.

Ainsi l'ensemble des procédés actuellement connus de synthèse de l'iodure de trifluorométhyle explique le coût extrêmement élevé de ce produit chimique.

L'industrie est donc toujours à la recherche d'un procédé économique de préparation de CF$_3$I, car ce composé a de nombreuses applications. En effet, les iodures de perfluoroalkyle sont des réactifs, très utiles pour la préparation d'alcools ou d'acides carboxyliques et sulfoniques polyfluorés.

Les iodures de perfluoroalkyle dont la chaîne alkyle contient un nombre pair d'atomes de carbone sont fabriqués à bas prix industriellement à partir de tetrafluoroéthylène matière première couramment disponible. Par contre, le 1er terme, l'iodure de trifluorométhyle n'est fabriqué qu'en très faible quantité par des techniques, comme décrit précédemment, très difficiles.

La présente invention a pour objet un procédé sûr et économiquement valable de préparation de l'iodure de trifluorométhyle à partir du bromure de trifluorométhyle qui lui est un gaz disponible sur le marché des produits chimiques car utilisé comme gaz extincteur dans l'électronique.

La présente invention a plus particulièrement pour objet un procédé de préparation de CF$_3$I par introduction dans une première étape soit d'un métal choisi parmi le zinc, le cadmium, l'aluminium, le manganèse avec du dioxyde de soufre soit de dithionite alcalin dans un solvant aprotique polaire, puis addition de bromure de trifluorométhyle, suivi après filtration dans une deuxième étape par l'addition d'iode dans un acide carboxylique ou sulfonique éventuellement perfluoré. La pression réactionelle dans la 1ère étape supérieure à 1 bar (10$^{-5}$Pa) avantageusement au plas égale à 50 bars (5.10$^6$Pa).

Le procédé de la présente invention n'utilise aucune matière première coûteuse ou dangereuse et permet l'obtention facile d'iodure de trifluorométhyle malgré de faibles rendements.

L'ordre d'introduction des réactifs est essentiel dans le cadre de la présente invention. En effet, la réaction n'est pas réalisable si l'on inverse l'ordre d'introduction des réactifs est essentiellement l'ordre d'introduction du dioxyde de soufre et du bromure de trifluorométhyle.

Parmi les métaux on préfère utiliser le zinc.

Parmi les solvants aprotiques polaires, on préfère utiliser les amides tels que notamment : le formamide, le diméthylformamide, la N-méthylpyrrolidone, le diméthylacétamide et des solvants tels que le sulfolane. On préfère tout particulièrement utiliser le diméthylformamide.

Parmi les dithionites alcalins on peut notamment citer le dithionite de sodium ou de potassium. On préfère utiliser le dithionite de sodium.

L'iode est ajouté en solution dans tout acide carboxylique ou sulfonique qui ne réagit pas avec le milieu réactionnel. Ainsi on préfère utiliser les acides carboxyliques ou sulfoniques contenant 1 à 4 atomes de carbone. Ces acides peuvent être eventuellement perfluorés. On préfère néanmoins utiliser l'acide acétique.

Selon le premier mode de mise en œuvre de l'invention lorsque l'on utilise un métal et du dioxyde de soufre et du bromure de trifluorométhyle, on introduit dans le réacteur de préférence un rapport molaire du bromure de trifluorométhyle au métal d'au moins 2 et un rapport molaire de dioxyde de soufre au métal compris de préférence entre 1 et 3. Le bromure de trifluorométhyle mis en excès peut être recyclé.

La quantité de solvant mise en œuvre est telle qu'il y ait de préférence entre 0,25 et 1 atome gramme de métal par litre de solvant.

De préférence on ajoute au dithionite une base choisie parmi les hydroxydes alcalins, alcalinoterreux, l'ammoniaque, les sels d'acides faibles tels que par exemple le phospate disodique, le métabisulfite de sodium, l'hydrogénosulfite de sodium, le borate de sodium.

Selon le deuxième mode de mise en œuvre de l'invention, lorsque l'on utilise un dithionite alcalin, on ajoute de préférence une base choisie parmi les hydroxydes alcalins, alcalinoterreux, l'ammoniaque, les sels d'acides faibles tels que par exemple le phosphate disodique, le métabisulfite de sodium, l'hydrogénosulfite de sodium, le borate de sodium.

On préfère utiliser le phosphate disodique.

Selon un procédé préféré de mise en oeuvre de l'invention le dithionite alcalin est introduit dans le réacteur en solution saturée dans l'eau ou le formamide. Il est même possible d'introduire le dithionite en partie sous forme solide et en partie en solution dans l'eau sous forme d'une suspension saturée. On préfère éliminer tout l'oxygène présent dans le réacteur puis on introduit le perhalogénométhane gazeux.

En ce qui concerne les conditions de réactions il est préférable de travailler à une température comprise entre 45° et 85°C et encore plus préférentiellement comprise entre 65° et 75°C.

La pression réactionnelle utilisée dans le 1ère étape selon le procédé est toujours supérieure à 1 bar ce qui est une condition essentielle lorsque l'on travaille avec un gaz peu soluble dans le solvant de réaction. Une pression comprise entre 1 et 50 bars est préférée bien que la limite supérieure ne soit pas un critère essentiel mais uniquement préférentiel du point de vue technique.

A la fin de la première étape réactionnelle réalisée selon le 1er ou 2ème mode de mise en oeuvre, on filtre la suspension obtenue, on élimine le dioxyde de soufre éventuellement introduit puis on ajoute l'iode dans un solvant carboxylique ou sulfonique éventuellement perfluoré.

L'iode est utilisé en quantité au moins stoéchiométrique calculée par rapport au métal ou au dithionite, un rapport molaire compris entre 0,5 et 2 est tout à fait préférentiel. Il est préférable de travailler à une témpérature comprise entre 100 et 140°C pour cette 2ème étape.

L'acide carboxylique ou sulfonique a pour fonction de solubiliser l'iode et de maintenir le pH du milieu réactionnel inférieur ou égal à 5. Il sera introduit en quantité nécessaire pour atteindre ce pH.

De préférence le réacteur ne doit pas être constitué d'un matériau ou métal pouvant réagir avec le dioxyde de soufre. On préfère ainsi utiliser un réacteur en verre.

Lors de la 2ème étape, après introduction de l'iode dans l'acide carboxylique ou sulfonique, l'iodure de trifluorométhyle se dégage instantanément et est récupéré dans un piège refroidi.

L'invention va être plus complètement décrite à l'aide des exemples suivants qui ne doivent pas être considérés comme limitatifs de l'invention.

EXEMPLE 1 :

Dans un flaçon en verre épais on place :
- 6,5 g de zinc,
- 4 g d'hydroxyde de sodium en poudre,
- 100 ml de diméthylformamide.

Le flaçon est placé dans un appareil de Parr. Après avoir fait le vide, on ajoute :
- 10 g de dioxyde de soufre.

La pression est ensuite portée à 3,7 bars par adjonction de bromotrifluorométhane. On agite le mélange pendant 2 heures. Le flaçon est alors ouvert, les solides sont éliminés par filtration, le dioxyde de soufre restant est évacué sous vide. On ajoute :
- 50 ml d'acide acétique glacial et
- 15 g d'iode.

Le mélange est porté à 120°C pendant 9 heures. Il se produit un dégagement gazeux. Les gaz sont récupérés dans un piège refroidi par de la carboglace puis distillés pour donner :
- 6,3 g d'iodure de trifluorométhane (Eb -22°C);
Rendement : 32 %.

EXEMPLE 2 :

Dans un flacon en verre épais on place :
- 10 g de dithionite de sodium dihydraté,
- 8 g d'hydrogénophosphate de sodium,
- 15 ml d'eau,
- 10 ml de diméthylformamide.

Le flacon est placé dans un appareil de Parr. Après avoir fait le vide dan le flacon, il est thermostaté à 65°C.

La pression est ensuite portée à 3,7 bars par adjonction de bromotrifluorométhane. On agite le mélange pendant 2 heures. Le flacon est alors ouvert, les solides sont éliminés par filtration, l'eau est évaporée sous vide, le diméthylformamide est récupéré est les cristaux formés sont rincés avec deux fois 10 ml de diméthylformamide. On ajoute :
- 50 ml d'acide acétique au diméthylformamide et,
- 15 g d'iode.

Le mélange est porté à 120°C pendant 3 heures. Il se produit un dégagement gazeux. Les gaz sont récupérés dans un piège refroidi par de la carboglace puis distillés pour donner :
- 0,6 g d'iodure de trifluorométhane ;
Rendement : 6 %.

**Revendications**

1 °) Procédé de préparation d'iodure de trifluorométhyle, caractérisé en ce que dans une 1ère étape, on introduit soit un métal chois parmi le zinc, le cadmium, l'aluminium, le manganèse avec du dioxyde de soufre soit du dithionite alcalin dans un solvant aprotique polaire suivi par l'addition de bromure de trifluorométhyle puis après filtration dans une 2ème étape par l'addition d'iode en solution dans un acide carboxylique ou sulfonique éventuellement perfluoré. La pression réactionnelle dans la 1ère étape étant supérieure à 1 bar $10^5$ $P_a$), avantageusement au plus égal à 50 bars ($5.10^6$ $P_a$).

2 °) Procédé selon la revendication 1, caractérisé en ce que le métal choisi est le zinc.

3 °) Procédé selon la revendication 1, caractérisé en ce que le solvant aprotique polaire est le diméthylformamide.

4 °) Procédé selon la revedication 1, caractérisé en ce que le dithionite alcalin est le dithionite de sodium.

5 °) Procédé selon la revendication 1, caractérisé en ce que l'acide carboxylique est l'acide acétique.

6 °) Procédé selon les revendications 1 et 2, caractérisé en ce que le rapport molaire du bromure de trifluorométhyle à l'atome gramme de métal est supérieur ou égal à 2.

7 °) Procédé selon les revendications 1 et 2, caractérisé en ce que le rapport molaire du dioxyde de soufre à l'atome gramme de métal est compris entre 1 et 3.

8 °) Procédé selon les revendications 1 et 2, caractérisé en ce que l'on met en oeuvre 0,25 à 1 atome gramme de métal par litre de solvant.

9 °) Procédé selon la revendication 1, caractérisé en ce que le dithionite est utilisé en présence d'une base ou d'un sel d'acide faible.

10 °) Procédé selon la revendication 1, caractérisé en ce que le dithionite est utilisé en présence de phosphate disodique.

11 °) Procédé selon la revendication 1, caractérisé en ce que le rapport molaire de l'iode au nombre d'atome gramme de métal ou au nombre de moles de dithionite est comprise entre 0,5 et 2.

## Patentansprüche

1. Verfahren zur Herstellung von Trifluorjodmethan, dadurch gekennzeichnet, daß man in einem ersten Verfahrensschritt entweder ein Metall, das Zink, Cadmium, Aluminium oder Mangan ist, mit Schwefeldioxid, oder Alkalidithionit in ein aprotisches polares Lösungsmittel gibt, anschließend Bromtrifluormethan hinzufügt und nach der Filtration in einem zweiten Verfahrensschritt in ggf. perfluorierter Carbon- oder Sulfonsäure gelöstes Jod zugibt. Der Reaktionsdruck im ersten Verfahrensschritt ist größer als 1 bar ($10^5$ Pa), vorzugsweise nicht höher als 50 bar ($5.10^6$ Pa).

2. Verfahren nach Anspruch 1, dadurch gekennzeichnet, daß das gewählte Metall Zink ist.

3. Verfahren nach Anspruch 1, dadurch gekennzeichnet, daß das aprotische polare Lösungsmittel Dimethylformamid ist.

4. Verfahren nach Anspruch 1, dadurch gekennzeichnet, daß das Alkalidithionit Natriumdithionit ist.

5. Verfahren nach Anspruch 1, dadurch gekennzeichnet, daß die Carbonsäure Essigsäure ist.

6. Verfahren nach den Ansprüchen 1 und 2, dadurch gekennzeichnet, daß man Schwefeldioxid verwendet und das Molverhältnis von Trifluorbrommethan zu Grammatom des Metalls größer oder gleich 2 ist.

7. Verfahren nach den Ansprüchen 1 und 2, dadurch gekennzeichnet, daß das Molverhältnis von Schwefeldioxid zu Grammatom des Metalls zwischen 1 und 3 liegt.

8. Verfahren nach den Ansprüchen 1 und 2, dadurch gekennzeichnet, dass man 0,25–1 Grammatom Metall zu einem Liter Lösungsmittel gibt.

9. Verfahren nach Anspruch 1, dadurch gekennzeichnet, daß das Dithionit in Anwesenheit einer Base oder eines Salzes einer schwachen Säure verwendet wird.

10. Verfahren nach Anspruch 1, dadurch gekennzeichnet, daß das Dithionit in Anwesenheit von Dinatriumhydrogenphosphat verwendet wird.

11. Verfahren nach Anspruch 1, dadurch gekennzeichnet, daß das Molverhältnis von Jod zu Grammatom des Metalls oder zur Molzahl des Dithionits zwischen 0,5 und 2 liegt.

## Claims

1. Process for the preparation of trifluoromethyl iodide, characterized in that, in a 1st stage, either a metal chosen from amongst zinc, cadmium, aluminium and manganese, with sulphur dioxide, or an alkali metal dithionite is introduced into a polar aprotic solvent, followed by the addition of trifluoromethyl bromide, followed, after filtration, in a 2nd stage, by the addition of iodine dissolved in a carboxylic or sulphonic acid which may be perfluorinated if required. The reaction pressure is the 1st stage being greater than 1 bar ($10^5$ Pa), advantageously at least equal to 50 bars ($5 \times 10^6$ Pa).

2. Process according to claim 1, characterized in that the metal chosen is zinc.

3. Process according to claim 1, characterized in that the polar aprotic solvent is dimethylformamide.

4. Process according to claim 1, characterized in that the alkali metal dithionite is sodium dithionite;

5. Process according to claim 1, characterized in that the carboxylic acid is acetic acid.

6. Process according to claims 1 and 2, characterized in that sulphur dioxide is used and the ratio of trifluoromethyl bromide in moles to metal in gram-atoms is greater than or equal to 2:1.

7. Process according to claims 1 and 2, characterized in that the ratio of sulphur dioxide in moles to metal in gram-atoms is between 1:1 and 3:1.

8. Process according to claims 1 and 2, characterized in that 0.25 to 1 gram-atom of metal is employed per litre of solvent.

9. Process according to claim 1, characterized in that the dithionite is employed in the presence of a base or of a weak acid salt.

10. Process according to claim 1, characterized in that the dithionite is employed in the presence of disodium phosphate.

11. Process according to claim 1, characterized in that the ratio of iodine in moles to metal in number of gram-atoms or to dithionite in number of moles is between 0.5:1 and 2:1.